# FASCICULE DE BREVET EUROPEEN

(11) **EP 0 507 687 B1**
(45) Date de publication et mention de la délivrance du brevet: **05.07.1995**
(21) Numéro de dépôt: 92400926.9
(22) Date de dépôt: 02.04.1992
(51) Int. Cl.: E03D 5/014, E03D 11/02, A47K 13/24

(54) **Ensemble sanitaire de toilettes pour séparer et collecter les urines radioactives**
Sanitärtoilette zur Trennung und Sammlung vom radioaktiven Urin
Sanitary toilet unit for separating and collecting radioactive urine

(30) Priorité: 04.04.1991 FR 9104103
(43) Date de publication de la demande: 07.10.1992
(73) Titulaire: MEDISYSTEM, F-78280 Guyancourt (FR)
(72) Inventeur: Delfosse, Jean, F-78370 Plaisir (FR)
(74) Mandataire: Dubois-Chabert, Guy

(56) Documents cités:
- DE-A- 1 609 239
- DE-C- 47 788
- DE-C- 634 420
- FR-A- 656 673

## Description

L'invention se rapporte aux équipements hospitaliers et, en particulier, aux lieux d'aisances destinés à des malades subissant des traitements radioactifs. L'invention concerne particulièrement une cuvette de toilettes ou de WC destinée à réceptionner les urines de manière séparée par rapport aux matières fécales comme dans DE-C-634,420.

Il est bien entendu connu d'utiliser dans les toilettes des cuves ou cuvettes de réception des urines et des matières fécales. Généralement, ces cuvettes sont accompagnées chacune d'un abattant permettant à l'utilisateur de s'asseoir sans avoir de contact direct avec la cuvette. Cette dernière est complétée d'un orifice principal d'évacuation des matières réceptionnées et d'un dispositif hydraulique de rinçage et d'évacuation de ces matières, appelée chasse d'eau. Ces cuvettes sont la plupart du temps en porcelaine, ou en métal léger.

Dans le milieu hospitalier, dans le cadre du traitement de certaines maladies ou de certains organes (par exemple, la glande thyroïde), il est devenu commun d'utiliser des thérapeutiques mettant en oeuvre ou utilisant des éléments radioactifs. Comme de plus, il a été constaté qu'une grande partie de cette radioactivité (environ 80 %) est expulsée par les urines, il est devenu impératif de traiter ces dernières de manière spéciale avant leur rejet dans un circuit d'évacuation relié directement au service public d'évacuation du type "tout-à-l'égout".

A cet effet, il a été déterminé et mis au point des systèmes de décroissance radioactive de ces urines contaminées dans des cuves spéciales. En effet, les urines sont stockées dans une ou plusieurs cuves pour permettre aux substances radioactives contenues dans les urines d'évoluer au cours du temps pour arriver en dessous d'un taux maximal déterminé de radioactivité. Les urines peuvent être alors rejetées dans les canalisations du réseau public d'évacuation. Cette manière de faire n'est applicable que dans la mesure où il est possible et relativement facile de collecter seules les urines des patients pour les stocker dans les cuves en question.

Le but de l'invention est de proposer un équipement capable de collecter facilement uniquement les urines des malades, lors de leur passage aux toilettes, sans que ceux-ci n'aient à faire de manoeuvre particulière pour évacuer les urines vers les cuves de décroissance.

A cet effet, l'objet principal de l'invention est un ensemble sanitaire de toilettes comportant une cuvette pour recueillir les urines et les matières fécales et comprenant :
- un abattant monté pivotant sur la cuvette de réception ;
- une première canalisation d'évacuation des matières fécales recueillies à l'arrière de la cuvette.

Selon l'invention, dans le but de réceptionner les urines de manière séparée par rapport aux matières fécales, la cuvette est partagée en un compartiment arrière destiné à recueillir les matières fécales et un compartiment avant destiné à recueillir les urines ; l'ensemble comprenant :
- une deuxième canalisation pour l'évacuation vers une cuve de stockage des urines réceptionnées dans le compartiment avant ;
- des moyens d'obturation de cette deuxième canalisation commandés de manière à fermer l'accès de cette deuxième canalisation pendant le rinçage, le lavage et l'entretien de la cuvette pour interdire aux produits de rinçage, de lavage et d'entretien l'accès à la deuxième canalisation. Les urines peuvent ainsi être évacuées séparément pour être éventuellement stockées et traitées suivant leur toxicité.

De manière préférentielle, l'ensemble selon l'invention comprend des moyens de commande de l'ouverture des moyens d'obturation, commandés par la descente de l'abattant sur la cuvette, grâce au poids de l'utilisateur qui s'asseoit sur l'abattant.

La deuxième canalisation n'est ainsi en communication avec la cuvette, que lorsque l'utilisateur est assis sur l'abattant. Son absence entraîne une fermeture automatique de cette deuxième canalisation.

La réalisation préférentielle de l'ensemble selon l'invention prévoit que les moyens d'obturation sont constitués d'un clapet de fermeture équipé d'une tige verticale de commande mobile en translation sous l'action de l'abattant pour ouvrir le clapet, un ressort étant prévu pour assurer la fermeture du clapet aussitôt que le poids de l'utilisateur n'est plus exercé sur l'abattant.

Dans sa réalisation préférentielle, les deux compartiments de réception sont séparés par une cloison.

La cloison possède un trou pour limiter le volume de liquide restant au-dessus du clapet fermé, dans le compartiment avant destiné à la réception des urines.

Dans une autre réalisation, les moyens de commande sont constitués d'une tige de commande reliée à l'abattant et agissant sur l'interrupteur d'un moteur électrique entraînant, par l'intermédiaire d'une tige de manoeuvre, la fermeture du clapet lorsqu'un utilisateur est assis sur l'abattant.

Il est avantageux de mettre une vanne de dérivation sur la deuxième canalisation pour permettre le prélèvement d'un échantillon d'urines.

### LISTE DES FIGURES

L'invention et ses différentes caractéristiques techniques seront mieux comprises à la lecture de la description détaillée qui suit et qui est annexée de différentes figures représentant respectivement :
- figure 1, le cadre de l'utilisation d'un ensemble sanitaire selon l'invention ;
- figure 2, l'ensemble sanitaire selon l'invention dans une première réalisation mécanique ;
- figure 3, le clapet et son système de commande dans la réalisation mécanique de la figure 2 de l'ensemble selon l'invention ;
- figure 4, une réalisation électromécanique de la commande du clapet dans l'ensemble selon l'invention.

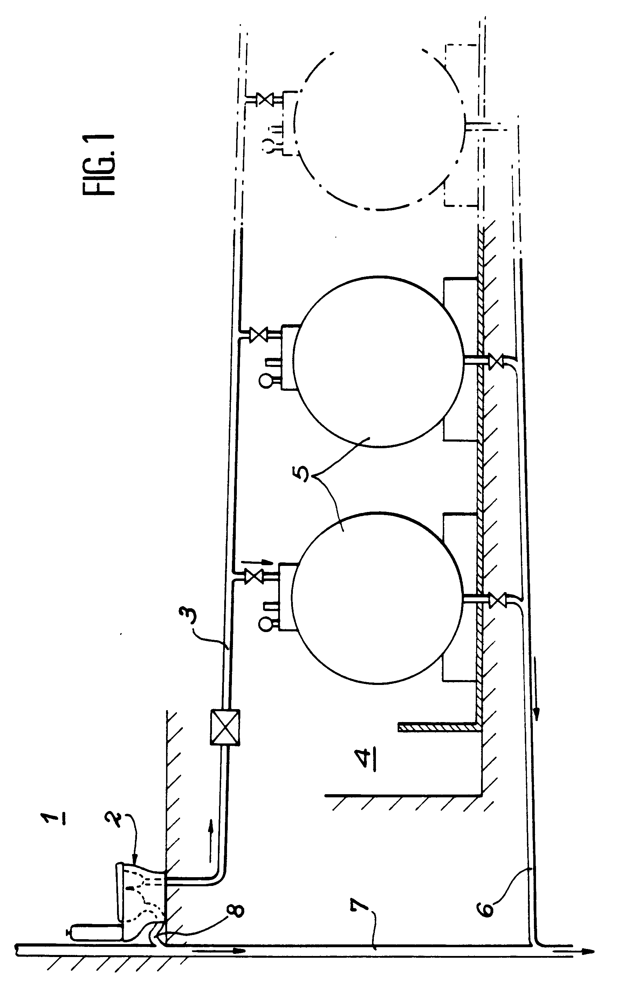

La figure 1 visualise les toilettes 1 ou les WC d'un hôpital ou d'une clinique dans lesquels est installé un ensemble sanitaire selon l'invention 2. Cet ensemble sanitaire 2 est relié par sa conduite principale d'évacuation 8 à une canalisation d'évacuation 7 reliée au circuit public d'évacuation du type "tout-à-l'égout". Par contre, une deuxième canalisation 3 relie cet ensemble sanitaire 2 à une batterie de cuves 5 placées dans un local spécialisé 4 destiné à la décroissance des urines contenant des particules radioactives.

Comme expliqué dans les paragraphes suivants, l'ensemble sanitaire selon l'invention permet de recueillir séparément les urines des patients subissant des traitements radioactifs et évacuant une grande partie de cette radioactivité dans leurs urines. Le stockage pendant un temps déterminé de ces urines est donc indispensable pour permettre le phénomène de décroissance. Le taux maximal de décroissance étant atteint, ces urines sont reversées dans le système d'évacuation central 7 au moyen d'une canalisation d'évacuation 6. Il est prévu au moins deux cuves de décroissance 5. En effet, il peut se trouver que la première cuve soit pleine avant que le taux de décroissance des urines qu'elle contient ne descende pas en-dessous du taux maximal accepté. Il est alors indispensable de continuer à stocker ces urines, et en même temps de procéder au stockage des nouvelles urines radioactives dans une autre cuve... , et ainsi de suite.

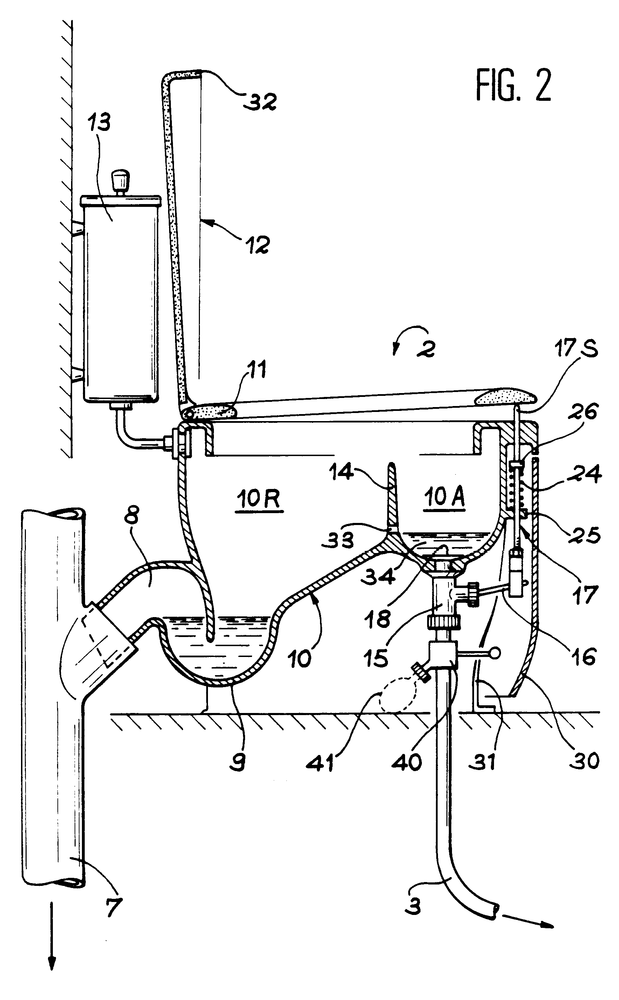

La figure 2 montre en coupe longitudinale l'ensemble sanitaire selon l'invention dans sa réalisation préférentielle. On retrouve les éléments principaux d'un ensemble sanitaire commun, c'est-à-dire une cuvette 10, un abattant sanitaire 11, monté pivotant sur celle-ci et destiné à recevoir un utilisateur, un couvercle 12 se rabattant sur l'ensemble et une chasse d'eau 13 pour le rinçage systématique de la cuvette 10. Cette dernière est reliée à une canalisation d'évacuation générale 7, par l'intermédiaire d'un siphon 9 et d'une première canalisation d'évacuation 8 par laquelle sont évacuées les matières fécales.

Selon l'invention, la cuvette 10 est partagée en un compartiment avant 10A et un compartiment arrière 10R. Etant entendu que tout utilisateur doit s'asseoir sur l'ensemble sanitaire pour l'utiliser, et en particulier sur l'abattant 11, on comprend aisément que les fonctions respectives de ces deux compartiments 10A et 10R sont de recueillir, pour le compartiment avant 10A les urines, et pour le compartiment arrière 10R les matières fécales.

L'ensemble est complété par une deuxième canalisation d'évacuation 3 reliée au fond du compartiment avant 10A. Comme le montre la figure 1, cette canalisation permet d'évacuer les urines vers les cuves de décroissance 5.

Le compartiment avant 10A est séparé du compartiment arrière 10R au moyen d'une cloison 14 permettant d'assurer, lors d'une utilisation de l'ensemble sanitaire, une réception complète de toutes les urines, uniquement dans le compartiment avant 10A.

Le compartiment avant 10A comprend des moyens d'obturation de cette deuxième canalisation d'évacuation 3 commandés par la présence d'un utilisateur sur l'ensemble sanitaire et plus précisément sur l'abattant 11. En effet, lorsque la personne utilisatrice est présente, il s'avère judicieux que la deuxième canalisation d'évacuation 3 soit en communication directe avec le compartiment avant 10A, c'est-à-dire que ces moyens d'obturation soient ouverts. Les urines peuvent ainsi être évacuées directement dans les cuves de décroissance. Par contre, il est indispensable que ces moyens d'obturation ferment l'accès à cette deuxième canalisation d'évacuation 3, lorsqu'il y a rinçage de la cuvette 10 au moyen du système de chasse d'eau 13, et lorsqu'on procède à l'entretien de sa cuvette 10 au moyen de différents produits, dont la présence doit être évitée dans les cuves de décroissance. Les moyens d'obturation doivent alors interdire l'accès à cette deuxième canalisation d'évacuation 3.

En conséquence, selon l'invention, les moyens d'obturation sont commandés à l'ouverture par la présence d'une personne sur l'abattant 11, et plus particulièrement par son poids lorsqu'elle s'asseoit sur l'abattant 11.

Comme le montre la figure 2, la réalisation préférentielle des moyens d'obturation prévoit l'utilisation d'un clapet 15 placé à l'entrée de cette deuxième canalisation d'évacuation 3, en bas du compartiment avant 10A. Ce clapet 15 est manoeuvré par une tige de manoeuvre 16 et une tige de commande 17 placée verticalement et faisant pression sur cette tige de manoeuvre 16.

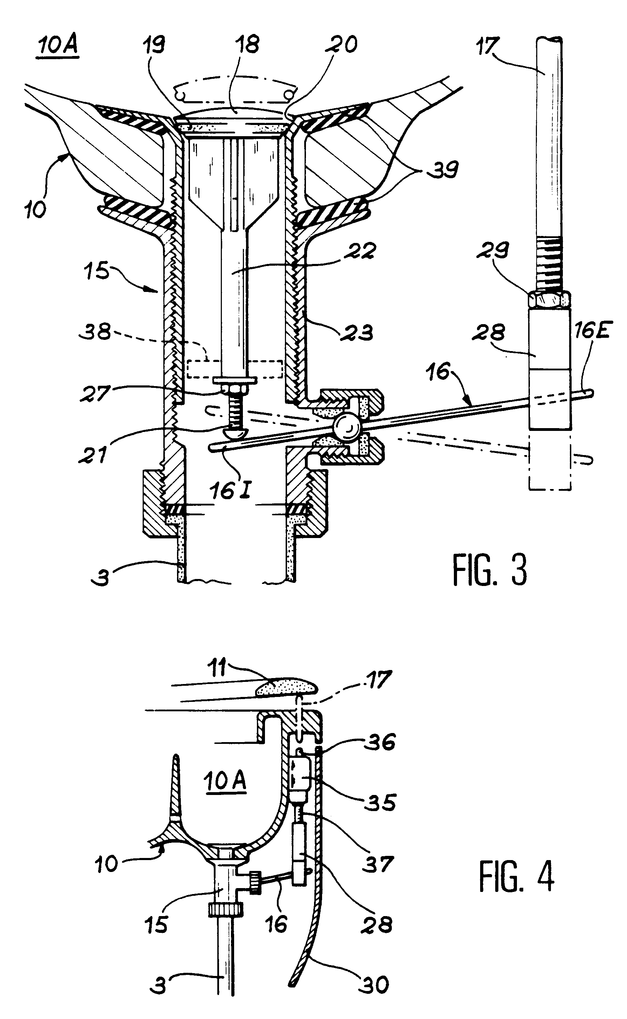

En se référant à la figure 3, on voit que la tige de manoeuvre 16 s'étend dans une direction à peu près horizontale lorsque le clapet 15 est placé verticalement. Une simple pression de haut en bas sur l'extrémité externe 16E de cette tige de manoeuvre 16 permet une ouverture de la bonde 18 du clapet 15. En effet, cette derniére est équipée d'un joint d'étanchéité circulaire 19 lui permettant de prendre appui sur une surface d'appui 20 de la partie inférieure du compartiment avant 10A. On note que ce type de clapet est utilisé couramment dans les lavabos vendus dans le commerce.

L'extrémité interne 16I de la tige de manoeuvre 16 est en appui en dessous d'une vis de réglage 21 vissée dans l'axe 22 du clapet 15 soutenant la bonde 18. La tige de manoeuvre 16 étant montée pivotante par rapport au corps 23 du clapet 22, au moyen d'un système à rotule, une pression de haut en bas sur l'extrémité externe 16E de la tige de manoeuvre 16 permet une remontée de l'axe 22 du clapet 15 et par conséquent, un soulèvement de la bonde 18, libérant ainsi l'accès à la deuxième canalisation d'évacuation 3. De plus, comme le montre la figure 2, l'extrémité supérieure 17S de la tige de commande 17 est en appui sous l'abattant 16, en bénéficiant d'un réglage adéquat de la longueur de cette tige de commande 17, une pression sur l'abattant 11 amène un basculement de la tige de manoeuvre 16 et l'ouverture du clapet 15.

Comme le montre la figure 2, un ressort 24 est monté autour de la tige de commande 17, prend appui par son extrémité inférieure sur un appui 25 solidaire de la cuvette 10. Il prend également appui par sa partie supérieure sur une rondelle 26 solidaire de la tige de commande 17. De ce fait, il exerce une force ascendante sur la tige de commande 17. Cette force est prévue de telle manière que, en l'absence d'une personne utilisatrice sur l'abattant 11, celui-ci se trouve soulevé d'une distance correspondant à la distance nécessaire à imprimer à l'extrémité externe 16E de la tige de manoeuvre 16 pour ouvrir le clapet 15. Toutefois, la force du ressort est prévue bien inférieure au poids minimal d'un utilisateur.

De ce fait, la présence d'une personne sur l'abattant 11 amène la descente de celui-ci contre la partie supérieure de la cuvette 10 et l'ouverture du clapet 15. Par contre, dès que la personne utilisatrice se relève, le ressort 24 ramène la tige de manoeuvre 16 dans sa position initiale et ferme automatiquement le clapet 15 par la redescente de la bonde 18 contre la surface d'appui 20 de la cuve 10.

Le réglage de la longueur de l'axe 22 du clapet 15 est obtenu au moyen de la vis de réglage 21 montée dans celui-ci, un écrou de serrage 27 permettant de fixer ce réglage. De même, la liaison de la tige de commande 17 sur la tige de manoeuvre 16 se fait au moyen d'un manchon 28 se vissant sur la tige de commande 17, un écrou de serrage 29 permettant de fixer ce réglage.

Comme le montre la figure 2, il est préférable de prévoir un capot avant 30 de la lunette 10, pour éviter aux utilisateurs et aux personnes d'entretien de détériorer ce mécanisme de commande. Un regard peut toutefois être aménagé dans ce capot 30, où ce dernier peut être monté amovible par rapport à la cuvette 10. Il est également préférable de prévoir un pied d'appui avant 31 pour assurer la stabilité et le soutien de l'ensemble sanitaire selon l'invention.

Dans le cas où des prélèvements d'urines s'avèreraient nécessaires, une vanne de dérivation 40, commandée manuellement par une infirmière, est montée sur la deuxième canalisation 3, après le clapet 15. Il est ainsi possible de mettre sur cette vanne de dérivation 40 un flacon 41, de préférence blindé, pour prélever les urines collectées dans le compartiment avant 10A.

On constate ainsi que, une fois la personne utilisatrice debout, le clapet 15 ferme l'accès à la deuxième canalisation d'évacuation 3 et une partie de l'eau utilisée pour rincer la cuvette 10 et issue du système de chasse d'eau 13 peut rester dans ce premier compartiment avant 10A. On comprend que lors de la prochaine utilisation de l'ensemble sanitaire, la personne utilisatrice s'assoira sur l'abattant 11 et provoquera l'ouverture du clapet 15 et l'évacuation du volume 34 de liquide restant dans le compartiment avant 10A. Or, le but à atteindre à l'aide de l'ensemble sanitaire selon l'invention est d'éviter la présence importante de liquide et d'eau dans les cuves de décroissance. En pratiquant un ou plusieurs trous 33 dans la cloison 14 séparant les deux compartiments avant 10A et arrière 10R, à une hauteur proche de la partie inférieure de la cuvette 10, il est ainsi possible de limiter le niveau de liquide du volume 34 restant au-dessus de la bonde 18 du clapet 15. En effet, une fois le système de chasse d'eau 13 actionné, la quantité de liquide se trouvant dans le compartiment avant 10A s'écoulera en quelques secondes par l'orifice 33 dans le compartiment arrière 10R et se dirigera vers le siphon 9. Seule une quantité négligeable 34 d'eau restera dans le compartiment avant 10A. Un ou plusieurs trous 33 peuvent donc être pratiqués dans la cloison 14. On peut également envisager que celle-ci soit moins importante en hauteur tout en gardant une efficacité suffisante pour garder les urines d'un patient, lors de l'utilisation de l'ensemble sanitaire selon l'invention.

En se reportant à la figure 3, le clapet 15 peut être monté sur la cuvette 10 par l'intermédiaire de deux joints circulaires 39.

De plus, le guidage de l'axe 22 dans le clapet 15 est facilité par la présence d'une bague 38 comportant des trous pour filtrer grossièrement les urines.

On prévoit également que le couvercle 12 ait un rebord 32 recouvrant complètement l'abattant 11 et la partie supérieure de la cuvette 10 pour assurer une étanchéité supérieure de l'ensemble sanitaire vis-à-vis de la pièce dans laquelle il se trouve.

Sur la figure 4, la tige de commande 17 n'agit pas directement sur la tige de manoeuvre 16. En effet, un moteur de commande 35 peut être interposé et être commandé par la tige de commande 17, au moyen d'un interrupteur 36 placé en-dessous de celle-ci. Ce moteur 35 peut actionner la tige de manoeuvre 16 au moyen d'un arbre de sortie 37 fileté à l'intérieur du manchon 28. De cette manière, l'ouverture et la fermeture du clapet sont également asservies à la présence d'un utilisateur sur l'abattant 11.

De manière à éviter que l'opération de rinçage au moyen du système de chasse d'eau ne soit commandé trop prématurément, il est possible d'interdire le déclenchement de ce système de chasse d'eau par la position rabattue de l'abattant 11. Effectivement, ce dernier peut comporter des moyens annihilant la commande du système de chasse d'eau 13. Ceci permet d'éviter que la cuvette 10 ne soit rincée, alors que la bonde 18 du clapet 15 est encore en position soulevée.

De même, il est possible d'envisager une commande hydraulique du clapet 15, le moyen hydraulique remplaçant alors le moteur 35 de la réalisation de la figure 4.

Il est bien entendu que l'intérieur de la cuvette 10 doit recevoir un revêtement permettant un entretien et une décontamination réguliers et empêchant l'attaque de l'eau de javel ou des produits d'entretien sur la cuvette 10. Il s'agit en particulier du compartiment avant 10A, de la bonde 18 du clapet 15 et de l'intérieur de la deuxième canalisation d'évacuation 3. L'ensemble sanitaire selon l'invention ne nécessite aucune commande voulue ni spéciale, lors de chacune de ces utilisations, puisque la commande s'effectue automatiquement à l'aide de l'abattant 11 sur laquelle la personne utilisatrice s'assied.

D'autres systèmes de commande du clapet 15 peuvent être envisagés en étant commandés par la présence d'une personne sur l'abattant.

## Revendications

1. Ensemble sanitaire de toilettes (2) comportant une cuvette (10) pour collecter les urines et les matières fécales, et comportant :
- un abattant (11) monté pivotant par rapport à la cuvette (10) ;
- une première canalisation d'évacuation (8) pour l'évacuation des matières fécales recueillies à l'arrière de la cuvette (10),
une cuvette (10) qui est partagée en un compartiment arrière (10R) destiné à recueillir des matières fécales et un compartiment avant (10A) destiné à recueillir des urines ; l'ensemble comprenant :
- une deuxième canalisation d'évacuation (3) pour évacuer vers des cuves de stockage (5) les urines réceptionnées dans le compartiment avant (10A) ;
- des moyens d'obturation de cette deuxième canalisation d'évacuation (3) commandés pour fermer l'accès à cette deuxième canalisation d'évacuation (3) durant le lavage, le rinçage et l'entretien de la cuvette (10) pour interdire aux produits de rinçage, de lavage et d'entretien, l'accès à la deuxième canalisation d'évacuation (3).

2. Ensemble sanitaire selon la revendication 1, caractérisé en ce qu'il comprend des moyens de commande de l'obturation de la deuxième canalisation d'évacuation (3) commandés par la descente de l'abattant (1) sur la cuvette (10) grâce au poids de la personne utilisatrice qui s'assoit sur l'abattant (11).

3. Ensemble sanitaire selon la revendication 2, caractérisé en ce que les moyens d'obturation sont constitués d'un clapet (15) commandé par une tige verticale de commande (17) reliée à l'abattant (11) pour être ouvert lorsque la personne utilisatrice est assise sur l'abattant (11), un ressort (24) étant prévu pour assurer la remontée de l'abattant (11) et la fermeture du clapet (15), lorsque le poids de la personne ne s'exerce plus sur l'abattant (11).

4. Ensemble sanitaire selon l'une quelconque des revendications précédentes, caractérisé en ce que les deux compartiments avant (10A), arriére (10R) étant séparés par une cloison (14), cette cloison (14) possède au moins un trou (33) pour limiter le volume de liquide restant au-dessus du clapet (15) lorsqu'il est fermé.

5. Ensemble sanitaire selon la revendication 2, caractérisé en ce que les moyens de commande sont complétés par un moteur électrique (35) commandés au moyen d'un interrupteur (36) par la tige de commande (17) et agissant sur une tige de manoeuvre (16) du clapet (15) lorsqu'une personne utilisatrice est assise sur l'abattant (11).

6. Ensemble sanitaire selon l'une quelconque des revendications prédédentes, caractérisé en ce qu'il comprend une vanne de dérivation (40) placée sur la deuxième canalisation (3), après le clapet (15), pour permettre le prélèvement d'un échantillon d'urines dans un flacon (41).

## Claims

1. Sanitary unit for toilets (2) comprising a bowl (10) for collecting urine and faecal matter, and comprising:
- a toilet seat (11) mounted to pivot with respect to the bowl (10);
- a first discharge duct (8) for discharging the faecal matter collected at the rear of the bowl (10),
a bowl (10) which is split into a rear compartment (10R) for collecting the faecal matter and a front compartment (10A) for collecting urine; the unit comprising:
- a second discharge duct (3) for discharging towards the storage tanks (5) urine which has been received in the front compartment (10A);
- means for closing this second discharge duct (3), controlled to close off the access to this second discharge duct (3) during the washing, rinsing and cleaning of the bowl (10) in order to deny rinsing, washing and cleaning products access to the second discharge duct (3).

2. Sanitary unit according to Claim 1, characterized in that it comprises means for controlling the closure of the second discharge duct (3) which are controlled by the lowering of the toilet seat (1) over the bowl (10) by way of the weight of the user who sits on the toilet seat (11).

3. Sanitary unit according to Claim 2, characterized in that the closure means are formed by a flap valve (15) which is controlled to be open by a vertical control rod (17) connected to the toilet seat (11) when the user is sitting on the toilet seat (11), a spring (24) being provided to ensure that the toilet seat (11) is raised again and that the flap valve (15) is closed, when the weight of the user no longer bears on the toilet seat (11).

4. Sanitary unit according to any one of the preceding claims, characterized in that since the two front (10A) and rear (10R) compartments are separated off by a wall (14), said wall (14) has at least one aperture (33) for limiting the volume of the liquid remaining above the flap valve (15) when it is closed.

5. Sanitary unit according to Claim 2, characterized in that in addition the control means have an electric motor (35) controlled via a switch (36) by the control rod (17) and acting on an actuating rod (16) of the flap valve (15) when a user is sitting on the toilet seat (11).

6. Sanitary unit according to any one of the preceding claims, characterized in that it comprises a diverting valve (40) located on the second duct (3), downstream of the flap valve (15), to allow the taking of a sample of urine in a flask (41).

## Patentansprüche

1. Sanitärtoiletteneinheit (2) mit einem Becken (10) zum Sammeln von Urin und von Fäkalien, umfassend:
- eine Brille (11), schwenkbar angebracht in bezug auf das Becken (10);
- eine erste Entleerungsleitung (8) zur Entleerung der hinten im Becken (10) angesammelten Fäkalien, wobei dieses Becken (10) unterteilt ist in ein hinteres Abteil (10R) zur Aufnahme der Fäkalien und ein vorderes Abteil (10A) zur Aufnahme des Urins; dabei umfaßt die Einheit:
- eine zweite Entleerungleitung (3) zum Entleeren des in dem vorderen Abteil (10A) aufgenommenen Urins in Lagerfässer (5);
- Verschlußeinrichtungen dieser zweiten Entleerungsleitung (3), gesteuert um den Zugang zu dieser zweiten Entleerungsleitung (3) während des Waschens, des Spülens und des Unterhalts des Beckens (10) zu schließen, um die Spül-, Wasch- und Unterhaltsprodukte daran zu hindern, in die zweite Entleerungsleitung (3) einzudringen.

2. Sanitärtoiletteneinheit nach Anspruch 1, dadurch gekennzeichnet, daß sie Verschlußeinrichtungen der zweiten Entleerungsleitung (3) umfaßt, betätigt durch das Absenken der Brille (11) auf das Becken (10) durch das Gewicht der benutzenden Person, die sich auf die Brille (11) setzt.

3. Sanitärtoiletteneinheit nach Anspruch 2, dadurch gekennzeichnet, daß die Verschlußeinrichtungen gebildet werden durch eine Klappe (15), gesteuert durch eine vertikale Betätigungsstange (17), verbunden mit der Brille (11), um geöffnet zu sein, wenn die benutzende Person auf der Brille (11) sitzt, wobei eine Feder (24) vorgesehen ist, um das Wiederanheben der Brille (11) sicherzustellen und den Verschluß der Klappe (15), wenn das Gewicht der Person nicht mehr auf die Brille (11) wirkt.

4. Sanitärtoiletteneinheit nach einem der vorhergehenden Ansprüche, dadurch gekennzeichnet, daß die beiden Abteile, das vordere (10A) und das hintere (10R), getrennt sind durch eine Wand (14), wobei diese Wand (14) wenigstens ein Loch (33) aufweist, um das über der Klappe (15) verbleibende Flüssigkeitsvolumen zu begrenzen, wenn sie geschlossen ist.

5. Sanitärtoiletteneinheit nach Anspruch 2, dadurch gekennzeichnet, daß die Betätigungseinrichtungen vervollständigt werden durch einen Elektromotor (35), gesteuert mittels eines Schalters (36) durch die Betätigungsstange (17) und auf eine Betätigungshebel (16) der Klappe (15) wirkend, wenn eine Person auf der Brille (11) sitzt.

6. Sanitärtoiletteneinheit nach einem der vorhergehenden Ansprüche, dadurch gekennzeichnet, daß sie ein Abzweigventil (40) umfaßt, angeordnet in der zweiten Leitung (3), nach der Klappe (15), um die Entnahme einer Urinprobe in eine Flasche (41) zu ermöglichen.
